# EUROPEAN PATENT SPECIFICATION

(11) **EP 2 193 115 B1**
(45) Date of publication and mention of the grant of the patent: **09.11.2011**
(21) Application number: 08829609.0
(22) Date of filing: 08.09.2008
(51) Int. Cl.: C07C 215/40, A61K 49/06, A61K 51/04

(54) **COMPOUND, DIAGNOSTIC AGENT, NUCLEAR MAGNETIC RESONANCE ANALYSIS METHOD, NUCLEAR MAGNETIC RESONANCE IMAGING METHOD, MASS SPECTROMETRY METHOD AND MASS SPECTROMETRY IMAGING METHOD**
VERBINDUNG, DIAGNOSTIKUM, KERNMAGNETRESONANZANALYSEVERFAHREN, KERNMAGNETRESONANZBILDGEBUNGSVERFAHREN, MASSENSPEKTROMETRISCHES VERFAHREN UND MASSENSPEKTROMETRISCHES BILDGEBUNGSVERFAHREN
COMPOSES, AGENT DE DIAGNOSTIC, PROCEDE D'ANALYSE PAR RESONANCE MAGNETIQUE NUCLEAIRE, PROCEDE D'IMAGERIE PAR RESONANCE MAGNETIQUE NUCLEAIRE, PROCEDE DE SPECTROMETRIE DE MASSE ET PROCEDE D'IMAGERIE PAR SPECTROMETRIE DE MASSE

(30) Priority: 07.09.2007 JP 2007232659; 05.09.2008 JP 2008228439
(43) Date of publication of application: 09.06.2010
(73) Proprietor: CANON KABUSHIKI KAISHA, Ohta-ku Tokyo 146-8501 (JP)
(72) Inventor: YOSHIMURA, Kimihiro, Tokyo 146-8501 (JP); YAMAUCHI, Fumio, Tokyo 146-8501 (JP); KUGE, Katsuaki, Tokyo 146-8501 (JP); YANO, Tetsuya, Tokyo 146-8501 (JP); SHIRAKAWA, Masahiro, Kyoto-shi Kyoto 615-8510 (JP); AOYAMA, Yasuhiro, Kyoto-shi Kyoto 615-8510 (JP)
(74) Representative: Weser, Wolfgang
(86) International application number: PCT/JP2008/066495
(87) International publication number: WO 2009/031712

(56) References cited:
- WO-A-98/57578
- WO-A-2006/082108
- RENSHENG LUO ET AL: "NMR assignment of the R2 domain of pneumococcal choline binding protein A (CbpA)" JOURNAL OF BIOMOLECULAR NMR, KLUWER ACADEMIC PUBLISHERS, DO, vol. 32, no. 1, 1 May 2005 (2005-05-01), pages 93-93, XP019249567 ISSN: 1573-5001
- INBAR, LIVIA ET AL: "Metabolic regulation in Streptomyces parvulus during actinomycin D synthesis, studied with carbon-13- and nitrogen-15-labeled precursors by carbon-13 and nitrogen-15 nuclear magnetic resonance spectroscopy and by gas chromatography-mass spectrometry" JOURNAL OF BACTERIOLOGY, vol. 170, no. 8, 1988, pages 4055-4064, XP002511236

## Description

### TECHNICAL FIELD

The present invention relates to compounds labeled with stable isotope atoms. More specifically, the present invention relates to labeled choline in which the nitrogen atom of the quaternary ammonium and carbon atoms of all the methyl groups attached to this nitrogen atom are substituted with respective stable isotope atoms and labeled choline derivatives which are synthesized using the labeled choline. In addition, the present invention relates to a diagnostic agent containing these compounds. Furthermore, the present invention relates to a nuclear magnetic resonance analysis method, a nuclear magnetic resonance imaging method, a mass spectrometry method and a mass spectrometry imaging method using these compounds.

### BACKGROUND ART

In the field of biochemistry and the area of clinical laboratory examinations, tracing and detecting *in vivo* kinetics of biological substances such as biologically active substances, transmitter substances or nutrients or test agents has become more important. In such tracing and detecting, a specific atom in the molecular structure of the biological substances or test agents has been substituted with either one of isotope atoms selected from radioisotope atoms or stable isotope atoms thereof and such substituted molecules (labeled compounds) have been used for tracing and detecting.

A labeled compound having a radioisotope atom as an isotope atom enables to trace the behavior of the labeled compound with good sensitivity even in a living body by detecting emitted radioactive rays. In late years, these labeled compounds are of increased importance as in positron emission tomography (PET) and single photon emission computer tomography (SPECT). As labeled compounds for use in these test methods, compounds labeled with radioisotope atoms such as ¹¹C, ¹⁵O, ¹³N and ¹⁸F are used in PET, and compounds labeled with radioisotope atoms such as ^{99m}Tc and ¹²³I are used in SPECT. For example, testing technique in which in vivo kinetics of ¹⁸F-labeled glucose (FDG) is traced for diagnosing various diseases has been already commonly utilized in PET. In addition to FDG, use of ¹⁸F-labeled choline and ¹¹C-labeled muscarinic acetylcholine in PET is exemplified, for example, in Japanese Patent No. 2809145 and Japanese Patent Application Laid-Open No. H11-152270.

Another labeling technique with an isotope atom is a method using a stable isotope atom. Examples of stable isotope atoms usable as a label include deuterium (D: natural abundance ratio = 0.015%), ¹³C (natural abundance ratio = 1.1%), ¹⁵N (natural abundance ratio: 0.366%) and ¹¹O (natural abundance ratio = 0.038%). Since these stable isotope atoms do not emit radioactive rays, technique as mentioned above cannot be applied to detection and analysis with these atoms.

Because the number of neutrons in these stable isotope atoms is different from corresponding dominant nuclides, the mass number of the atom in itself as well as the nuclear spin is different from corresponding dominant nuclide. Therefore, techniques for selectively detecting a labeled compound containing stable isotope atoms include mass spectrometry which uses the difference in molecular weight and nuclear magnetic resonance (NMR) which uses the difference in the nuclear spin. Compounds in which hydrogen atom H is substituted with deuterium D are also used as labeled compounds because the change of the vibration mode in a molecule can be detected by infrared spectroscopy and Raman spectroscopy. For *in vivo* kinetics, nuclear magnetic resonance imaging (MRI) method is commonly used to trace and detect a labeled compound with a stable isotope atom.

### DISCLOSURE OF THE INVENTION

There are the following problems when a compound labeled with a radioisotope atom is used.

Among radioisotope atoms, F, Tc and I are elements which are not or hardly found in a living body. Therefore, in case that the compounds labeled with these atoms are used to trace *in vivo* kinetics of a certain biological compound, they may not exhibit the same behavior as a substance which inherently occurs in a living body. The above-mentioned FDG is used as a labeled compound of glucose, which is an important energy source in a living body. Since FDG has a similar molecular structure of a non-labeled compound, it is taken into biotissues. However, the subsequent metabolic process of FDG is different from glucose.

In the meantime, oxygen, carbon and nitrogen are basic atoms constituting a biological substance. Therefore, labeled compounds in which these radioisotope atoms (¹¹C, ¹³N, ¹⁵O) are used may exhibit similar behavior *in vivo* as the non-labeled compound, as long as the molecular structure of the labeled compounds are same as that of a compound which originally occurs in a living body.

In addition, there is a problem to be solved in handling radioactive substances as shown below. First of all, expensive facilities such as cyclotron are necessary to generate radioisotope atoms. Also many of radioactive nuclides have short life periods, and there has been limitation in time period allowed from generation of radioactivity atoms to labeling of the target compound and for a series of examination from administration to a test subject to measurement. There is also a problem to be solved that the test subject and people who perform test and measurement may be exposed to radioactive rays emitted from these radioisotope atoms.

On the other hand, in case of using compounds labeled with stable isotope atoms, there are problems and difficulties to be solved described below.

Since the stable isotope atoms are present in certain constant ratios in nature, when mass spectrometry of a molecule consisting of hydrogen, carbon, nitrogen and oxygen is performed, the obtained spectrum is not a single one but a complicated pattern referred to as isotopic distribution depending on the contents of D, ¹³C, ¹⁵N and ¹⁷O. The pattern becomes markedly complicated as the number of atoms constituting the biological substance increases, and analysis becomes difficult. For example, in ¹³C-NMR, which is a commonly utilized analysis technique, influence by ¹³C present in substances other than the labeled compound cannot be neglected (the natural abundance ratio of carbon atom ¹³C is around 1.1%).

Substitution of hydrogen atom ¹H with deuterium is widely utilized for stable isotope labeling, while the deuterium substitution has the following problems to be solved. First of all, exchange reaction between hydrogen and deuterium may occur at substituted positions. The exchange reaction is liable to occur particularly in an aqueous solution in which protons are present as in a living body. Therefore, the expected effect cannot be provided by the labeling with deuterium in an aqueous solution. In addition, NMR measurement is generally performed mainly based on proton NMR (¹H-NMR) measurement mode and the information obtained from proton NMR decreases when hydrogen atom ¹H is substituted with deuterium. Furthermore, although there are measurement techniques based on various principles in mass spectrometry, it is necessary to ionize the substance to be measured by mass spectrometry. Since the ionization often relies on addition or elimination of protons, substitution of atoms other than hydrogen atom with the isotope is more preferable. Furthermore, in consideration of the influence by change in molecular weight resulted from addition or elimination of protons as well as complication of the peaks (in a range of isotopic distribution) due to stable isotope atoms occurring in nature and so on, it is desirable that the labeled compound has a molecular weight in mass spectrometry which is different from the molecular weight of the non-labeled compound by 3 mass or more.

In addition, another problem of utilizing a compound labeled with a stable isotope atom is lower detection sensitivity as compared with the technique utilizing a radioisotope. Therefore, recognition of the existence of the labeled compound utilizing a compound labeled with a stable isotope atom is difficult when the abundance of the labeled compound in a living body or in biotissues is very small. It is effective to substitute atoms of a biological substance or a test agent to improve the detection sensitivity of the labeled compound so that the ratio of the stable isotopes may largely exceed the ratio of the stable isotopes occurring in nature. Compounds in which carbon and/or nitrogen atoms of amino acids, nucleobases and glucose are substituted with ¹³C and/or ¹⁵N are commercially available. However, in the case that a ¹³C-substituted amino acid is used, even if a very small amount of ¹³C-substituted amino acid can be detected, it is difficult to discriminate whether the detected amino acid is an amino acid which contains naturally occurring stable isotope atom ¹³C or a ¹³C amino acid which has been administered as a test agent.

There is an idea to elevate sensitivity by increasing the dose. For example, if a F atom of ¹⁸FDG used in PET is changed to ¹⁹F having a nuclear spin as ¹⁹FDG, detection by nuclear magnetic resonance is theoretically enabled, but administration to a living body in an amount which is necessary for the detection by current nuclear magnetic resonance causes apprehension of toxic influence by FDG. On the other hand, if a biological substance labeled with a stable isotope atom ¹³C, ¹⁵N or ¹¹O is used as a test agent, toxicity can be reduced. However, for the present, there are only limited compounds such as ¹¹O-substituted water and oxygen molecule (Japanese Patent Application Laid-Open No. 2000-004491), ¹³C-substituted glucose (Japanese Patent Application Laid-Open No. 2006-256962) and ¹³C-substituted deoxyglucosone derivatives (Japanese Patent No. 2727289) as biological substances which can be administered in an amount sufficient for actually enabling analysis/test by NMR and mass spectrometry.

In addition, even if a substance substituted with a stable isotope atom is a biological substance, the amount which can be administered is limited, and there are few compounds whose acceptable dose can satisfy detection sensitivity of NMR and mass spectroscopy. Furthermore, even if there are biological substances whose allowable dose can satisfy detection sensitivity, compounds whose *in vivo* kinetics are worth testing/analyzing have been extremely limited.

Summarizing the above, the problems to be solved by the present invention are (1) to select biological substances which are worth testing/analyzing; (2) to produce labeled compounds of the selected biological substances not using a radioisotope atom which has a risk of radioactive exposure and limitation in handling time but using a stable isotope atom; and (3) that the labeled compound can be discriminated from natural compounds of the selected biological substances which are substituted with stable isotope atoms and that the compound can be measured with good sensitivity.

Accordingly, the present inventors have conducted intensive studies on the above problems to be solved, and consequently have found that by selecting choline as the object to be examined and by labeling choline by substituting the nitrogen atom of the quaternary ammonium group and all the carbon atoms attached to the quaternary ammonium group with respective stable isotope atoms ¹⁵N and ¹³C, the labeled choline and the non-labeled choline can be selectively detected separated from each other. In addition, the present inventors have newly synthesized derivatives of labeled choline and have found that they can also be used as stable isotope labeled compounds. These labeled compounds can be surely detected by multiple resonance measurement in NMR which utilizes magnetic coherence transfer between three elements of ¹H-¹³C-¹⁵N. Further, since four atoms are substituted, they can be detected surely separated from non-labeled compounds and naturally occurring compounds which are substituted with stable isotope atoms even by mass spectrometry.

Thus the compounds according to the present invention are compounds represented by the following general formula (1). 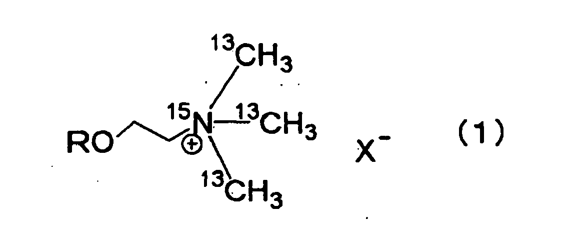 In general formula (1), R is an arbitrary monovalent group. Here, when R is a hydrogen atom, this compound is a labeled choline, and when R is other than a hydrogen atom, this compound is a labeled choline derivative.

Labeled choline is represented by general formula (1) wherein all of the carbon atoms of the methyl group and the nitrogen atom of the quaternary ammonium group are respectively isotopes ¹³C and ¹⁵N.

In addition, X⁻ is a monovalent anion in general formula (1). X⁻ functions as a counter anion. Specific examples of X⁻ may include a fluoride ion, a chloride ion, a bromide ion, an iodide ion, a hydroxide ion and a tartrate ion. Here, when R has a negative charge, there is no need for a counter anion and therefore X⁻may be absent.

In addition, the diagnostic agent according to the present invention is a diagnostic agent containing a compound represented by general formula (1) in a ratio which exceeds the natural abundance ratio in isotope labeled compounds of said compounds.

In addition, the nuclear magnetic resonance method according to the present invention is a nuclear magnetic resonance analysis method wherein presence of the compound represented by general formula (1) is recognized utilizing magnetic coherence transfer between nuclei in the ¹H-¹³C-¹⁵N bond of the compound.

In addition, the mass spectrometry method according to the present invention is a mass spectrometry method comprising a step of calculating the abundance ratio of the compound represented by general formula (1) to the other isotope compounds of the compound.

According to the present invention, labeled compounds for choline and choline derivatives using stable isotope atoms which can be surely detected by NMR and mass spectrometry can be obtained.

### BRIEF DESCRIPTION OF THE DRAWINGS

FIG. 1 is a one-dimensional proton NMR spectrum of labeled choline.
FIG. 2 is a triple resonance NMR spectrum of labeled choline.
FIG. 3 shows MS spectra of labeled choline and non-labeled choline.
FIG. 4 is a triple resonance NMR spectrum of labeled acetylcholine.
FIG. 5 shows MS spectra of labeled acetylcholine and non-labeled acetylcholine.
FIG. 6 is a triple resonance NMR spectrum of labeled phosphocholine.

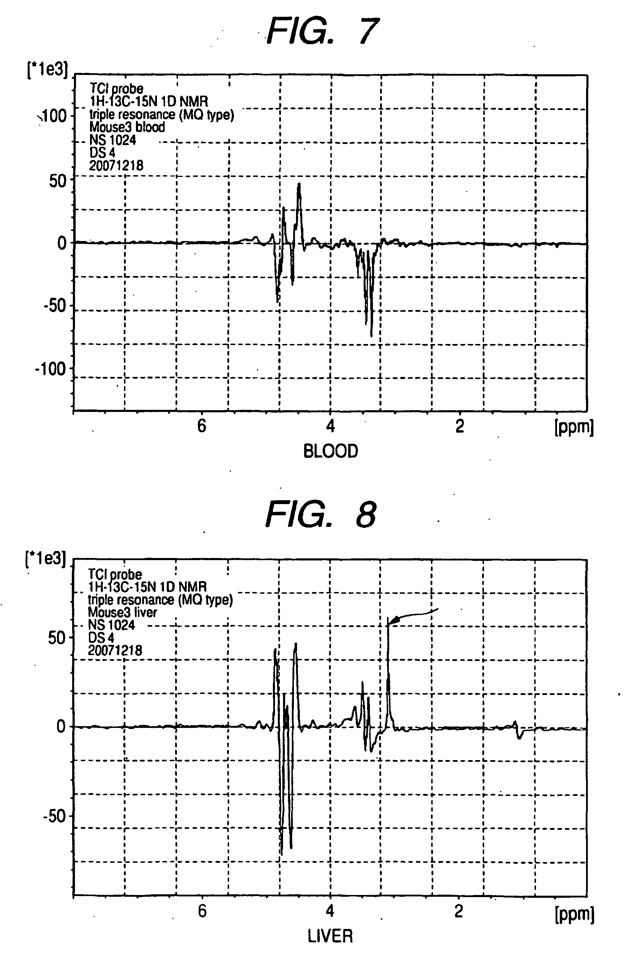

FIG. 7 is a triple resonance NMR spectrum of blood from cancer bearing mouse.
FIG. 8 is a triple resonance NMR spectrum of liver from cancer bearing mouse.

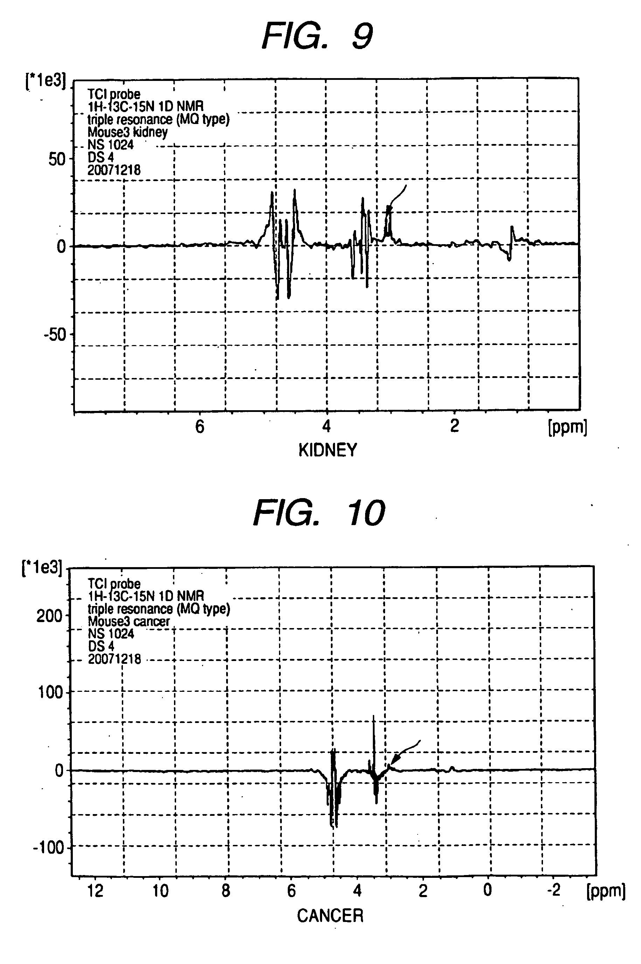

FIG. 9 is a triple resonance NMR spectrum of kidney from cancer bearing mouse.
FIG. 10 is a triple resonance NMR spectrum of cancer tissue from cancer bearing mouse.

### BEST MODES FOR CARRYING OUT THE INVENTION

The present inventors have conducted intensive studies on various biological substances with regard to acceptable dose to a body, accumulated amount in a body and detection sensitivity by a non-radioactive detecting device, and as a result decided to pay attention to choline. Choline is one of vitamin B complex, and can be widely found in animal and plant tissues not only in a free form but also in the form of phosphatidylcholine such as dihexadecylphosphatidylcholine (DHPC) and dipalmitoylphosphatidylcholine (DPPC), phospholipid such as sphingomyelin or in the form of acetylcholine. In this way, the compounds are present in a living body not only as choline by itself but also in the form of a plurality of choline derivatives, which means that choline taken into biotissues stays in a living body for some period of time in the forms bearing important roles and shows that there is a possibility that choline and choline derivatives can be detected and that the kinetics thereof are worth tracing. Here, choline is represented by the following general formula (4). 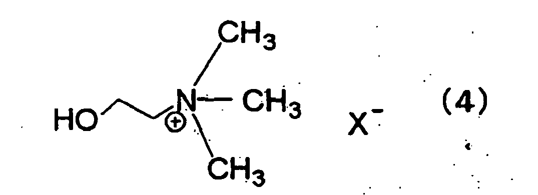 X⁻ is a monovalent anion. Specific examples of X⁻ may include a fluoride ion, a chloride ion, a bromide ion, an iodide ion, a hydroxide ion and a tartrate ion.

In addition, when choline and choline derivatives in which H of the hydroxyl group of choline is substituted are displayed together, it can be represented by the following general formula (5).

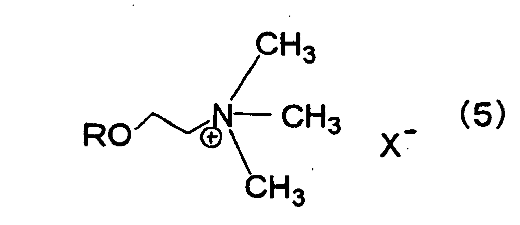

As for the action of choline in itself, it is known that choline has various kinds of actions such as regulation of fat metabolism, blood pressure-lowering effect and gastric secretagogue action.

In mammals, phosphatidylcholine is synthesized from food-derived choline. Specifically, choline is phosphorylated by adenosine triphosphate (ATP) to give phosphorylcholine, and phosphorylcholine reacts with cytidine triphosphate (CTP) to give CDP-choline. The phosphorylcholine part of CDP-choline is further transferred to diacylglycerol to give phosphatidylcholine. Phosphatidylcholine is also referred to as lecithin, and it is a phospholipid most highly contained in eukaryotic organisms. Phosphatidylcholine forms a lipid bilayer membrane and is the main component of cell membranes.

Furthermore, sphingomyelin is formed by the reaction of phosphatidylcholine and ceramide (N-acylsphingosine). Sphingomyelin can be found a lot in internal organs of animals, particularly in the brain and the nervous system and bears an important role on the metabolism and function of the brain and the nervous system.

Acetylcholine is known as another derivative synthesized from choline. Acetylcholine can be found a lot in biotissues, particularly in the nervous tissue, and it is an important chemical neurotransmitter substance along with adrenalin. Decrease in the intracerebral concentration of acetylcholine is supposed to be one of the causes of Alzheimer's disease. Acetylcholine is produced from acetyl-CoA and choline with a help of acetyltransferase in a living body.

As described above, choline is present as a number of choline derivatives in a living body while keeping the main part structure thereof, and administered choline has a high probability of remaining in a living body and biotissues, which means that it has a possibility to be detected in biotissues even with an analyzer having low detection sensitivity. Furthermore, choline derivatives play various important roles in a living body as described above. Therefore, significance to detect choline and choline derivative in a living body and biotissue is considered to be high. For example, it is known that choline kinase activity is increased in tumor cells which repeat abnormal cell division and that intracellular concentration of choline and derivatives thereof (phosphocholine and phosphoethanolamine) increases. That is, synthesis of cell membrane phospholipid mainly composed of phosphatidylcholine is activated. Since increase in choline metabolism has been recognized in prostate cancer, brain cancer and breast cancer, there is a large possibility as a diagnostic agent for detecting a cancer.

In addition, choline in itself is derived from food and is readily taken into cells because it is a raw material of the cell membrane. Existence in a living body in the form of choline by itself is known, and it is considered that there is some acceptable dose from the viewpoint of safety.

Based on the above considerations, the present inventors have conducted intensive studies on detecting choline and choline derivatives which are substituted with stable isotope atoms with high sensitivity at high resolution by detection technique which does not use radioactive rays.

First, substitution from ¹H to deuterium has been examined. When detection by nuclear magnetic resonance (NMR) is considered, the substitution of ¹H with deuterium is not preferred because the element which can be detected with the best sensitivity by NMR in the case of the same abundance is ¹H. Furthermore, when analysis by mass spectrometry is considered, the substitution of ¹H with deuterium is not preferred because the ionization process during mass spectrometry measurement is often accompanied with addition or elimination reaction of protons or hydrogen atoms and analysis of the measurement results may have become complicated if substituting ¹H with deuterium. In addition, phenomenon in which hydrogen atoms at some binding sites are easily substituted in the form of proton exchange in a living body can occur. Therefore, the inventors judged that significance of substituting hydrogen atom ¹H with deuterium D is small in labeling with a stable isotope atom when detection by mass spectrometry or nuclear magnetic resonance is assumed.

Next, substitution from ¹²C to ¹³C in the carbon atom has been examined. Molecular weight of choline increases depending on the number of the substitution to ¹³C carbon. In addition, carbon atoms in a molecule are not easily replaced with another carbon atom in a living body. Therefore, it is enabled by substituting some number (for example, 3) of carbon atoms with ¹³C to distinguish from originally existing choline by mass spectrometry. In addition, since the natural abundance ratio of ¹³C is about 1%, probability that there exists a molecule in which three carbon atoms are substituted with ¹³C is about 1/million, a considerably low probability which will occur in nature. However, when accumulated level of administered labeled choline in the target part in a living body is low, there is still a possibility that contrast against naturally occurring choline having ¹³C at the same position as the labeled choline is not enough.

On the other hand, when detection by nuclear magnetic resonance is considered, there is also a possibility that ¹³C-NMR measurement utilizing the nuclear spin of ¹³C is enabled by the substitution to ¹³C carbon. In addition, detection by multiple resonance utilizing magnetic coherence transfer between ¹H-¹³C is also possible when ¹H is attached to the substituted ¹³C. In multiple resonance detection, approximately the same sensitivity as in ¹H-NMR can be expected by detecting ¹H in itself which is attached to ¹³C. However, in consideration of natural abundance ratio of ¹³C, there is also a possibility that the state in which the abundance of ¹³C-labeled choline is a low cannot be distinguished from choline which contains naturally occurring ¹³C even when multiple resonance between ¹H-¹³C is utilized.

Next, among the elements which constitute choline, the nitrogen atom has been paid attention to. The nitrogen atom has also a stable isotope atom: ¹⁵N, and since the nuclear spin of ¹⁵N is I = 1/2, detection thereof by NMR is theoretically possible but the sensitivity of ¹⁵N-NMR is not high. In addiction, since the change in molecular weight of choline by ¹⁵N-substitution is only about one mass, it is difficult to effectively distinguish a ¹⁵N-substituted labeled choline compound from a naturally occurring ¹³C-substituted or D-substituted choline except when a device which can distinguish the change in molecular weight by ¹³C or D-substitution and the change in molecular weight by ¹⁵N-substitution is used utilizing a super-high resolution device.

Based on the above, it was considered that there was a possibility that the labeled compound having a single element substituted with a stable isotope was difficult to distinguish from choline which contained a naturally occurring stable isotope atom in NMR or mass spectrometry.

Therefore the present inventors have conducted further studies, and consequently have found that the selective detection with high sensitivity of the labeled compound can be detected by substituting the nitrogen atom of the quaternary ammonium group of choline and all the carbon atoms of the methyl groups attached to this nitrogen atom with respective stable isotopes.

Specifically, it has been found that labeled choline in which the nitrogen atom of the quaternary ammonium group of choline and all the carbon atoms of the three methyl groups attached to this nitrogen atom are selectively substituted with ¹⁵N and ¹³C, respective stable isotopes, and labeled choline derivatives obtained from the labeled choline could be detected with high sensitivity. Four stable isotope atoms are introduced in these compounds, and thus it has been enabled to remarkably reduce the possibility of having the same elemental composition as this based on the natural abundance ratio. This facilitates separated detection of choline and choline derivatives containing a naturally occurring stable isotope from the labeled choline and labeled choline derivatives in mass spectrometry.

Furthermore, in this case of four-atom substitution, that is, in the compounds (labeled choline or labeled choline derivatives) in which both of the nitrogen atom of the quaternary ammonium group of choline or choline derivatives and the carbon atoms of the methyl groups attached to this nitrogen atom are selectively substituted with ¹⁵N and ¹³C, respective stable isotope atoms, the compounds have a structure in which elements having I = 1/2 (¹H, ¹³C, ¹⁵N) are successively linked, which enables selective detection by nuclear magnetic resonance. In the case of this atomic arrangement, magnetic coherence transfer between nuclear spins of the ¹H-¹³C-¹⁵N bond enables triple resonance. In addition to that, all of ¹Hs are equivalent hydrogen atoms attached to a methyl group, and the number thereof is nine per a choline molecule. Thus, it has been found that labeled choline and labeled choline derivatives can be detected with high sensitivity surely distinguished from choline and choline derivatives which contain naturally occurring stable isotope atoms by detecting ¹H utilizing triple resonance between bonds in ¹H-¹³C-¹⁵N.

The labeled choline and labeled choline derivatives having stable isotope atoms as well as nuclear magnetic resonance utilizing them as labeled compounds or the mass spectrometry technique of the present invention stated above enable to examine the presence/absence of the labeled choline and labeled choline derivatives in a living body or in tissues. In addition, abundance ratio of non-labeled to labeled ones can be examined.

Here, labeled choline and labeled choline derivatives according to the present invention are compounds represented by the following general formula (1) :

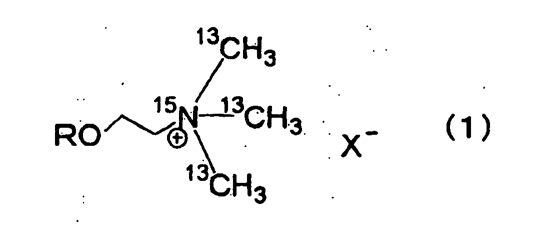

R is an arbitrary monovalent group and X⁻represents a monovalent anion in formula (1). X⁻functions as a counter anion. Specific examples of X⁻may include a fluoride ion, a chloride ion, a bromide ion, an iodide ion, a hydroxide ion and a tartrate ion. However, X⁻ may be absent when R has a negative charge.

In addition, labeled choline according to the present invention is a compound represented by the following general formula (2).

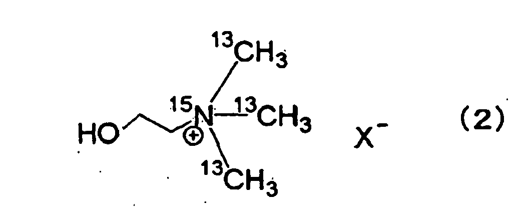

In addition, labeled choline derivatives according to the present invention are compounds represented by the above general formula (1) in which R is other than a hydrogen atom.

Specific examples of the labeled choline derivatives may include compounds represented by the following general formula (6). 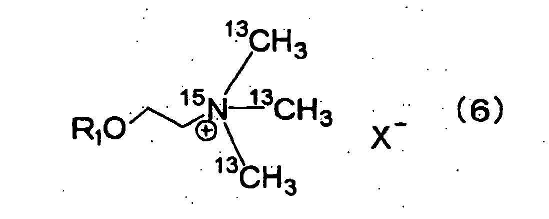 R₁ is an alkylcarbonyl group having 2 or more and 4 or less carbon atoms or R₁O is a phosphate residue in general formula (6).

Here, the compounds represented by this general formula (6) represent compounds represented by the above general formula (1) in which the R is an alkylcarbonyl group having 2 or more and 4 or less carbon atoms or the RO is a phosphate residue.

More specifically, labeled choline derivatives having a phosphate ester structure represented by the following general formula (3) can be mentioned. The following general formula (3) further specifies the case that R₁O is a phosphate residue in the above general formula (6). 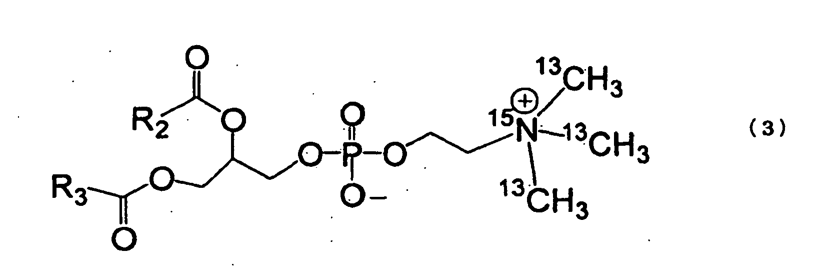 (wherein R₂ and R₃ each independently represents a saturated or unsaturated alkyl group having 10 or more and 20 or less carbon atoms.)

Specific examples of these labeled choline derivatives include compounds in which the nitrogen atom of the quaternary ammonium group and all the carbon atoms of the methyl groups attached to the nitrogen atom of acetylcholine, phosphocholine, phosphatidylcholine such as dihexadecylphosphatidylcholine (DHPC) and dipalmitoylphosphatidylcholine (DPPC), and sphingomyelin are substituted with respective stable isotope ¹⁵N or ¹³C_{.}

These compounds can be used as a diagnostic agent in the diagnosis which utilizes nuclear magnetic resonance and mass spectrometry. Specific examples of the diagnostic agents may include MR contrast agents and diagnostic agents for mass spectrometry.

In order to exhibit the function as diagnostic agents, the abundance of the compound represented by general formula (1) in the isotope compound group of diagnostic agents should exceed the natural abundance ratio in isotope labeled compounds of said compounds. In other words, if the ratio of labeled choline or labeled choline derivatives (these are the compounds represented by the above general formula (1)) in choline or choline derivatives (these are the compounds represented by the above general formula (5)) in a diagnostic agent does not exceed the natural abundance ratio, they cannot be distinguished from the background. In addition, the more the abundance of a compound represented by general formula (1) in the measurement system, the better, from a viewpoint to further improve the function as a diagnostic agent. As one of the techniques to increase abundance of the compound represented by general formula (1) in the measurement system, considered is to increase the ratio of the amount (number of molecules) of the compound represented by general formula (1) to the amount (number of molecules) of the compound represented by general formula (5) contained in the above diagnostic agent as much as possible. For example, it is preferable that the number of molecules of the compound represented by general formula (5) in the diagnostic agent is larger than the total number of molecules of all the other isotope compounds of the compound contained in the diagnostic agent. In addition, it is more preferable that the abundance (number of molecules) of a compound represented by general formula (1) in the isotope compound group in the diagnostic agent is 90% or more.

As for the method which can be used for the diagnosis utilizing these diagnostic agents, the following methods can be mentioned:
- A nuclear magnetic resonance analysis method wherein presence of the above compound is recognized utilizing magnetic coherence transfer between nuclei in the ¹H-¹³C-¹⁵N bond of the compound.
- A nuclear magnetic resonance imaging method wherein position where the above compound is present in a sample is recognized by the nuclear magnetic resonance analysis method utilizing magnetic coherence transfer between nuclei in the ¹H-¹³C-¹⁵N bond of the compound.
- A mass spectrometry method wherein the abundance ratio of the above compound and the other isotope compounds of the compound is calculated.
- A mass spectrometry imaging method wherein obtaining information on position where the above compound is present in a sample by calculating the abundance ratio of the above compound to the other isotope compounds of the compound.

In addition, the compounds of the present invention have a possibility to be utilized as a therapeutic drug as well.

Furthermore, the present inventors have conducted intensive studies on the technology to locate the position where the labeled choline and labeled choline derivatives are present in a living body or biotissues.

As a result, it has been found that it is possible to place a living body sample piece as a test sample on the specimen stage, irradiate it with a laser or ions such as Ga ion and analyze ion species released from the irradiated surface, for example, in mass spectrometry technique. Therefore, it has been found that mass spectrometry imaging technique which can locate the position in the sample where the labeled compound is present by mapping the parts where the labeled choline and labeled choline derivatives are present can be provided.

In nuclear magnetic resonance analysis technique as well, nuclear magnetic resonance imaging technique (so-called MRI) can theoretically make visible the position where the labeled choline and labeled choline derivatives are present by measuring multiple resonance which utilizes magnetic coherence transfer in the ¹H-¹³C-¹⁵N while applying a slice magnetic field to the living body.

Generally MRI detection is not highly sensitive. However, according to the present invention, the labeled compound can be detected with high sensitivity since presence of ¹³C and ¹⁵N having a low gyromagnetic ratio can be detected by ¹H having a high gyromagnetic ratio through combination with multiple resonance. Furthermore, in the case of the labeled choline and labeled choline derivatives of the present invention, since ¹H which can participate in multiple resonance between three elements of ¹H-¹³C-¹⁵N is present in the number as many as nine per molecule, there is a possibility that detection can be achieved with higher sensitivity as compared with labeled compounds commonly commercially available in the market so far.

### EXAMPLES

### (Example 1)

According to the following scheme, labeled choline in which all the carbon atoms of the methyl group and the nitrogen atom of the ammonium group were respectively substituted with ¹³C and ¹⁵N was synthesized. 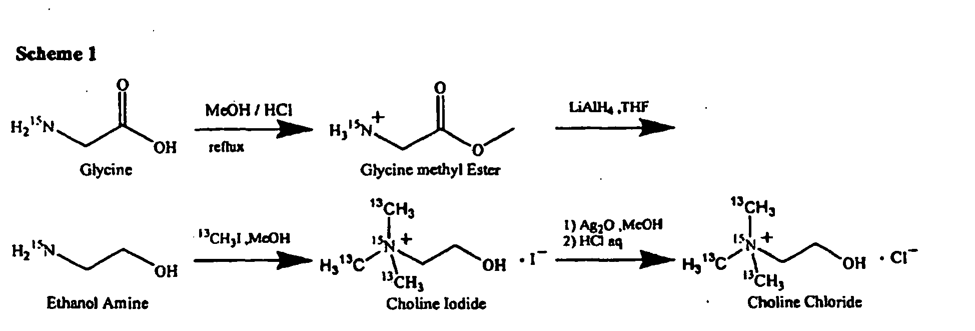 Specific synthesis procedure is shown in the following.

### <Synthesis of ¹⁵N-glycine methyl ester hydrochloride>

Hydrogen chloride generated by adding concentrated sulfuric acid (30 ml) to sodium chloride (31.097 g, 532 mmol) was blown into ice-cooled dry methanol (100 ml). ¹⁵N-glycine (76.07 mg, 1.00 mmol) was added to this and heated to reflux under argon atmosphere for one hour. The solvent was evaporated under reduced pressure, and white solid-shaped ¹⁵N-glycine methyl ester hydrochloride (1.24 g, 9.78 mmol, 98%) was obtained.

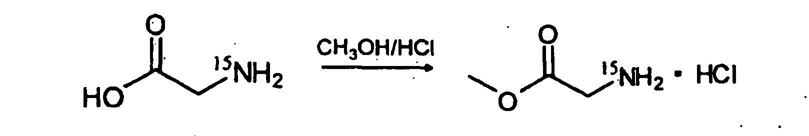

### <Synthesis of ¹⁵N-ethanolamine>

¹⁵N-glycine methyl ester hydrochloride (1.24 g, 9.78 mmol) synthesized in the above procedure and sodium hydrogen carbonate (1.92 g, 19.2 mmol) were suspended in tetrahydrofuran (250 ml) and stirred at room temperature for 30 minutes. Then lithium aluminum hydride (376 mg, 9.91 mmol) was added and stirred at ordinary temperature for ten hours. After that, distilled water (4 ml) was slowly added to deactivate excessive lithium aluminum hydride. The generated precipitation was removed by filtration and washed with dry methanol, and the solvent was evaporated from the filtrate and the rinsing liquid under reduced pressure. Dry methanol (20 ml) was added to the residue and the remaining solid was removed again. The solvent was evaporated under reduced pressure from the filtrate and ¹⁵N-ethanolamine (approximately quantitative) was obtained as a colorless transparent oil.

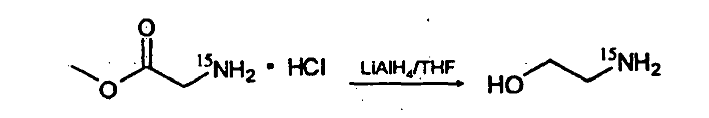

### <Synthesis of ¹³C₃-¹⁵N-choline iodide>

¹⁵N-ethanolamine (607 mg, 9.78 mmol) obtained in the above procedure was dissolved in dry methanol (25 ml), and after added with ¹³C-methane iodide (5.00 g, 34.9 mmol) and potassium carbonate (5.2 g, 37.62 mmol), stirred under argon atmosphere at ordinary temperature for ten hours. After insoluble inorganic salt was removed by filtration, the solvent was evaporated under reduced pressure, and ¹³C₃-¹⁵N-choline iodide was obtained (approximately quantitative) as a pale yellow solid.

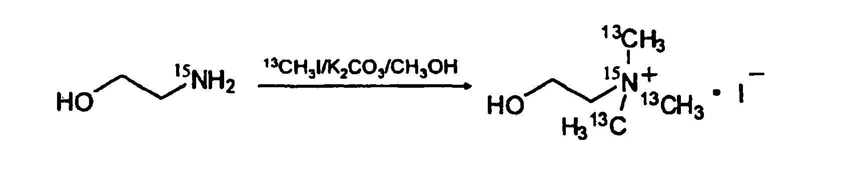

### <Synthesis of ¹³C₃-¹⁵N-choline chloride>

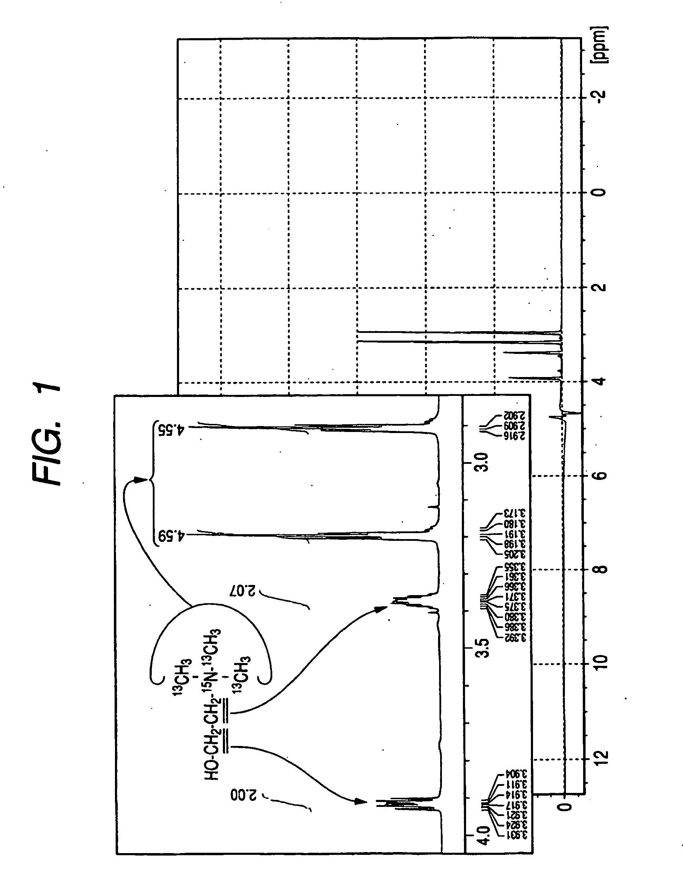

¹³C₃-¹⁵N-choline iodide (2270 mg, 9.78 mmol) synthesized in the above procedure was dissolved in methanol (30 ml) and after added with silver oxide (4.69 g, 20.2 mmol), stirred for 15 minutes, and solids were removed by filtration. 0.5M hydrochloric acid aqueous solution was added dropwise to the filtrate till pH became 4 and then the solvent was evaporated under reduced pressure. Dry ethanol (30 ml) was added to the residue and insoluble solids were removed by filtration. The solvent was evaporated under reduced pressure from the filtrate and ¹³C₃-¹⁵N-choline chloride was obtained (387 mg, 2.70 mmol, 27%) as a pale yellow solid.
¹H NMR (500 MHz, (ppm, D₂O): 2.91, 3.19 (together t, together 4.5H, CH₃), 3.35-3.39 (m, 2H, HOCH₂CH₂), 3.90-3.93 (m, 2H, HOCH₂CH₂) (See FIG. 1); MS (FAB) m/z 108 (M + H⁺)

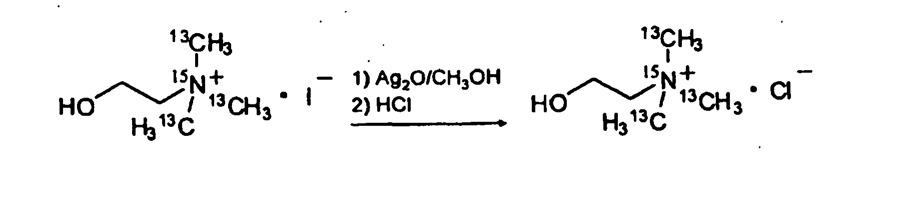

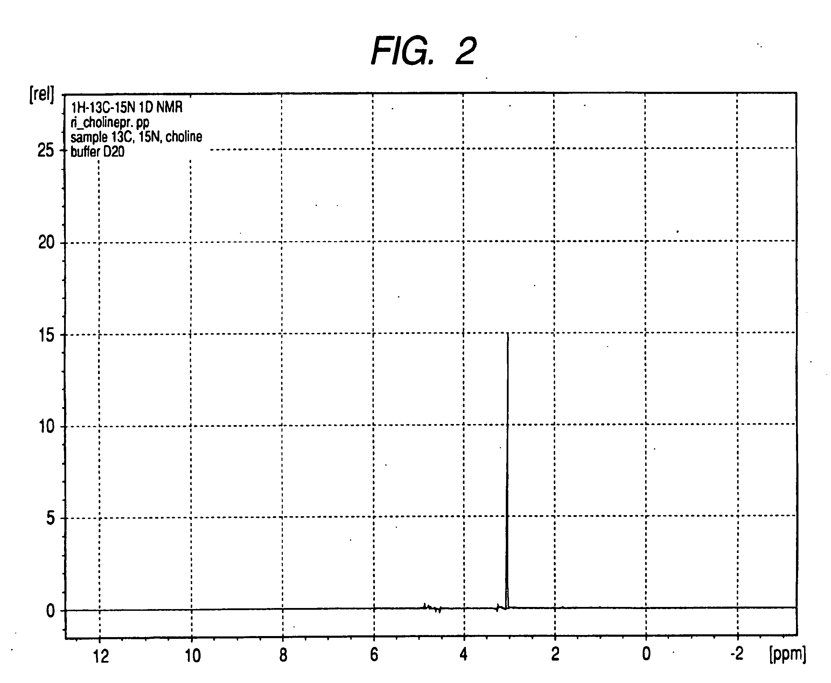

Multiple resonance measurement utilizing magnetic coherence transfer between three elements of ¹H-¹³C-¹⁵N was performed on the synthesized labeled choline, and protons derived from the methyl group were able to be selectively detected (FIG. 2).

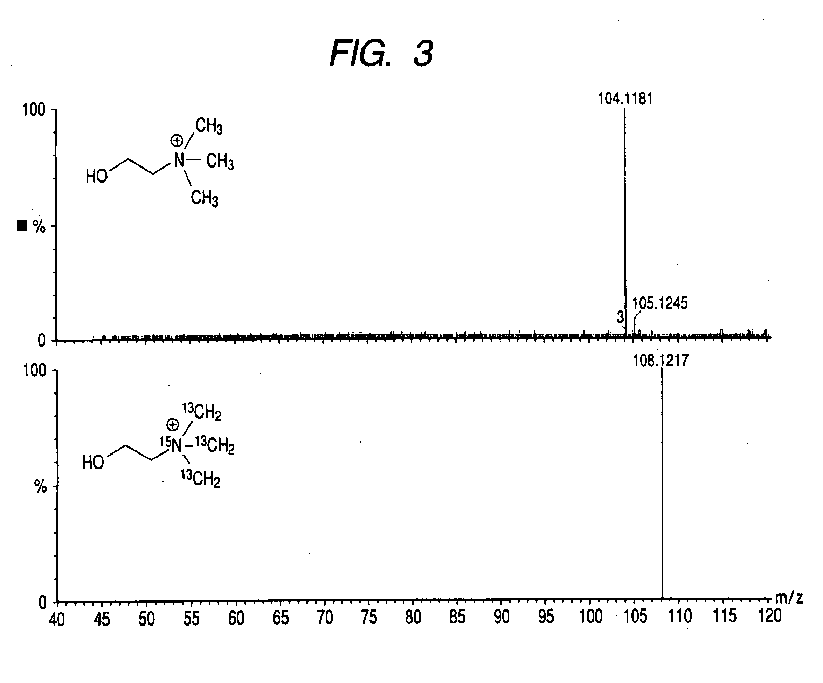

The synthesized labeled choline was further subjected to LC-ESI-MS measurement, and an exact Mass: 108.1217 (calculated value: 108.1146) corresponding to a composition formula: C₂¹³C₃H₁₄¹⁵NO was confirmed and thus it was confirmed that the isotopic labeling aimed at was achieved from the comparison with non-labeled choline (exact Mass: 104.1181 (calculated value: 104.1075) (FIG. 3).

### (Example 2)

According to the following scheme, labeled acetylcholine which was a labeled choline derivative was synthesized. 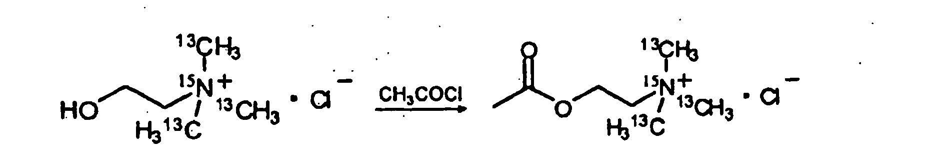 ¹³C₃-¹⁵N-choline chloride (27.9 mg, 0.194 mmol) synthesized in Example 1 was added to acetyl chloride (10 ml) and heated to reflux under argon atmosphere for one hour. The solvent was evaporated under reduced pressure and ¹³C₃-¹⁵N-acetylcholine chloride was obtained as a pale yellow solid (16.8 mg, 0.091 mmol, 47%).
¹H NMR (500 MHz, (ppm, D₂O): 2.00 (s, 3H, CH₃COO), 2.93, 3.22 (together t, together 4.5H, CH₃), 3.59-3.60 (m, 2H, HOCH₂CH₂), 4.40-4.43 (m, 2H, HOCH₂CH₂); MS (FAB) m/z 150 (M + H⁺)

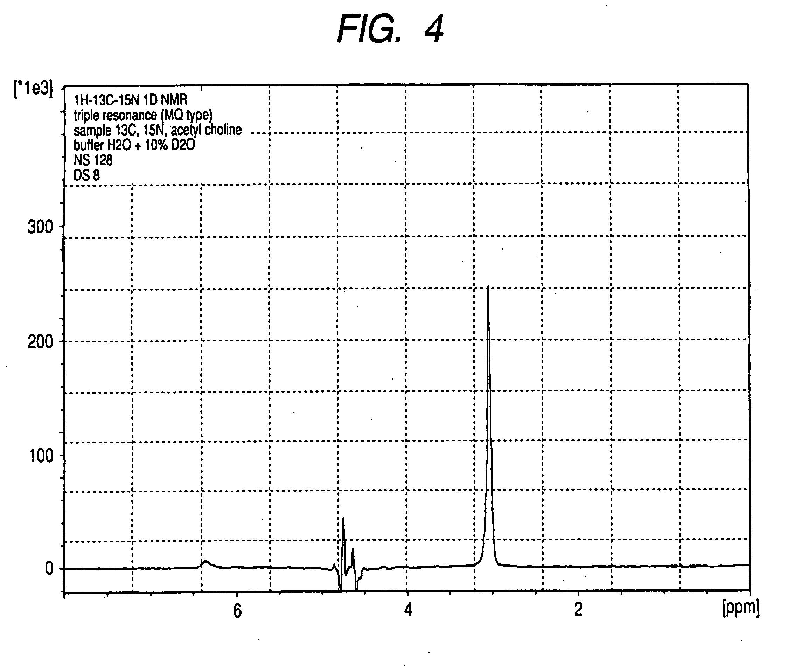

Multiple resonance measurement utilizing magnetic coherence transfer between three elements of ¹H-¹³C-¹⁵N was performed on the synthesized labeled acetylcholine, and protons derived from the methyl group was able to be detected at high intensity (FIG. 4).

LC-ESI-MS measurement of the synthesized labeled acetylcholine confirmed an exact Mass: 150.13 corresponding to a composition formula: C₄¹³C₃H₁₆¹⁵NO₂ and thus the difference in the molecular weight from non-labeled acetylcholine (comparison) was confirmed.

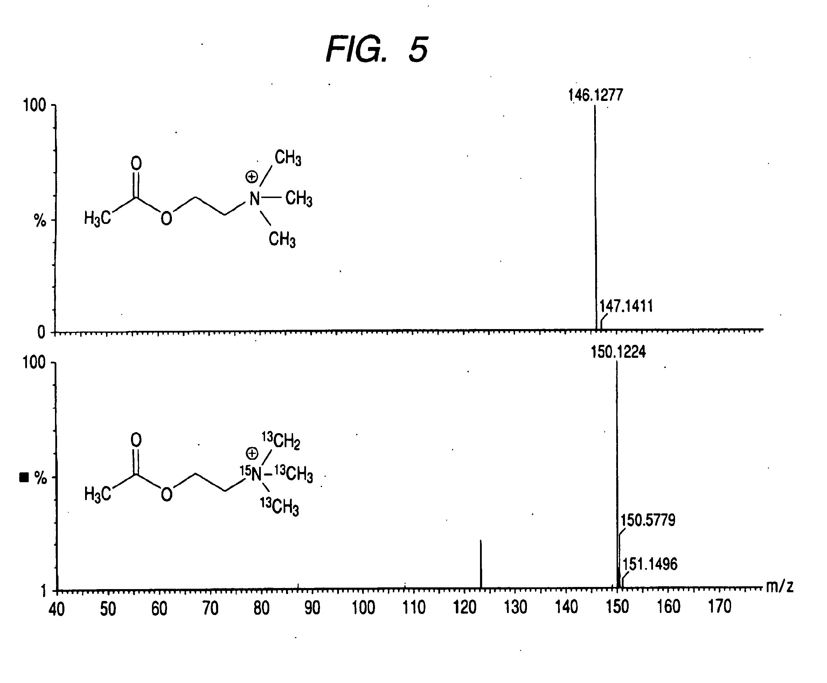

The synthesized labeled acetylcholine was further subjected to LC-ESI-MS measurement, and an exact Mass: 150.1224 (calculated value: 150.1252) corresponding to a composition formula: C₂¹³C₄H₁₆¹⁵NO was confirmed and thus it was confirmed that the isotopic labeling aimed at was achieved from the comparison with non-labeled acetylcholine (exact Mass: 146.1277 (calculated value: 146.1181) (FIG. 5).

### (Example 3)

According to the following scheme, labeled phosphocholine which was a labeled choline derivative was synthesized.

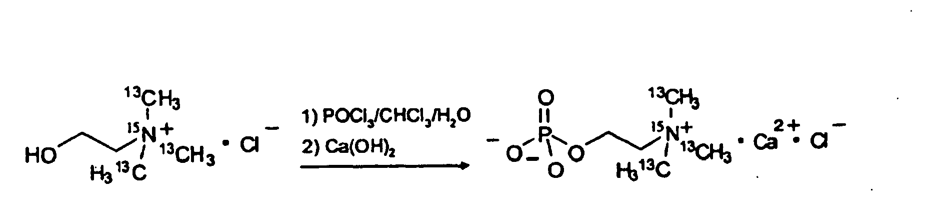

Chloroform (180 µl) and distilled water (20 µl) were added to phosphoryl chloride (175.5 mg, 1.15 mmol) and stirred at room temperature for 15 minutes. ¹³C₃-¹⁵N-choline chloride (20.1 mg, 0.141 mmol) synthesized in Example 1 was added to this and stirred at room temperature for 15 hours. After reaction, distilled water (150 µl) was added and the aqueous phase was separated. Calcium hydroxide was added to this till pH became 10 and after solids were removed by filtration, the filtrate was evaporated under reduced pressure, and phosphorylated ¹³C₃-¹⁵N-choline chloride was obtained as a calcium salt (approximately quantitative). ¹H NMR (500 MHz, (ppm, D₂O): 2.93, 3.22 (together t, together 4.5H, CH₃), 3.41-3.46 (m, 2H, POCH₂CH₂), 4.01-4.05 (m, 2H, POCH₂CH₂)

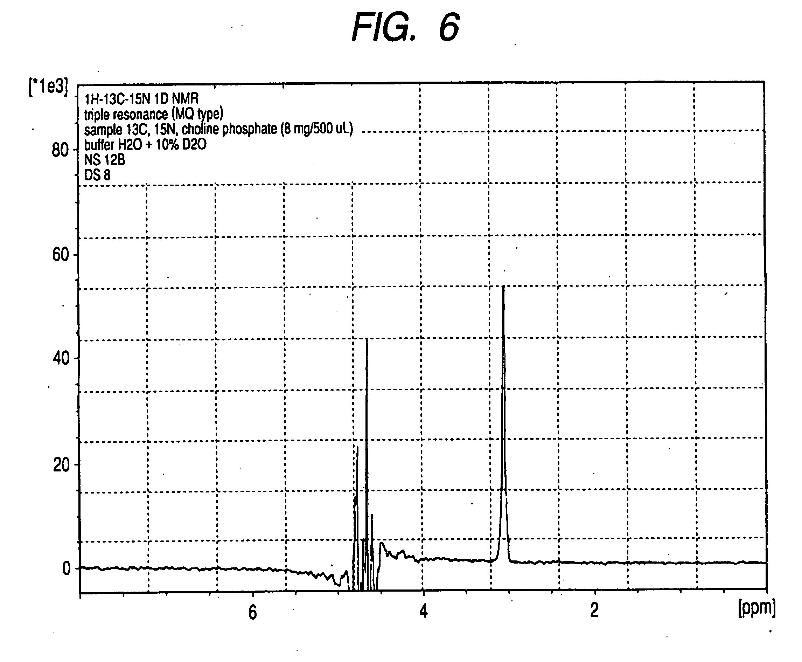

Multiple resonance measurement utilizing magnetic coherence transfer between three elements of ¹H-¹³C-¹⁵N was performed on the synthesized labeled phosphocholine, and protons derived from the methyl group were able to be selectively detected (FIG. 6).

LC-ESI-MS measurement of the synthesized labeled phosphocholine confirmed an exact Mass: 188.08 corresponding to a composition formula: C₂¹³C₃H₁₅¹⁵NO₄P and the difference in the molecular weight from non-labeled phosphocholine (comparison) was confirmed.

### (Example 4)

¹³C₃-¹⁵N-choline chloride synthesized in example 1 was administered to a cancer bearing mouse and ¹³C₃-¹⁵N-choline chloride in organs was detected by NMR.

### (i) Preparation of a cancer bearing mouse

Meth-A cells cultured in abdominal cavity of a BALB/c mouse were harvested by administrating 50 ml saline to the mouse. The cells were collected by centrifugation and resuspended in PBS (50 ml). The cells were counted and prepared at 2.0 × 10⁷/ml. 100 µl of the solution containing the cells was subcutaneously injected to a left foot of another BALB/c mouse.

### (ii) Administration of ¹³C₃-¹⁵N-choline chloride

One hundred micro litter of ¹³C₃-¹⁵N-choline chloride dissolved in PBS (2 mg/ml) was administrated to the cancer bearing mouse via the vein of the tail. After 1 hour, the mouse was dissected and the necessary organs (blood (200 µl), liver, kidney, cancer) were collected into microtubes. 1 × Lysis buffer (500 µl) was added to each of the tubes and the organs were mashed by using beads. The microtubes were centrifuged (14,000 rpm, 30 min) for the debris to be precipitated and the supernatants were collected and subjected to multiple resonance measurement utilizing magnetic coherence transfer between three elements of ¹H-¹³C-¹⁵N.

As the result, signals derived from methyl groups of cholines were detected at 3.0 ppm in liver, kidney and cancer.

### (Comparison)

### <NMR measurement>

Multiple resonance measurement utilizing magnetic coherence transfer between three elements of ¹H-¹³C-¹⁵N was performed on commercially available choline (non-labeled product), and protons derived from the methyl group were not able to be detected.

## Claims

1. A compound represented by the following general formula (1),
wherein R is an arbitrary monovalent group and X⁻represents a monovalent anion, provided that X⁻ may be absent when R has a negative charge.

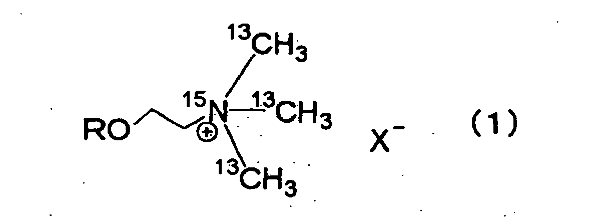

2. The compound according to claim 1 wherein the compound is a labeled choline having a structure represented by the following general formula (2).

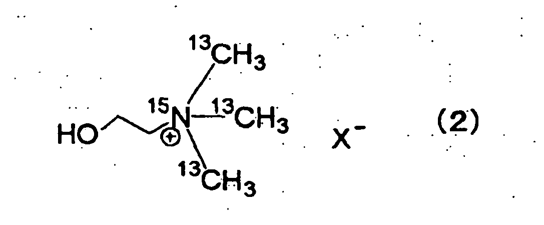

3. The compound according to claim 1 wherein the R is other than a hydrogen atom.

4. The compound according to claim 3 wherein the R is an alkylcarbonyl group having 2 or more and 4 or less carbon atoms or the RO is a phosphate residue.

5. The compound according to any of claims 1 to 4 wherein the X⁻ is any one of a fluoride ion, a chloride ion, a bromide ion, an iodide ion, a hydroxide ion and a tartrate ion.

6. The compound according to claim 4 wherein the compound has a structure represented by the following general formula (3), 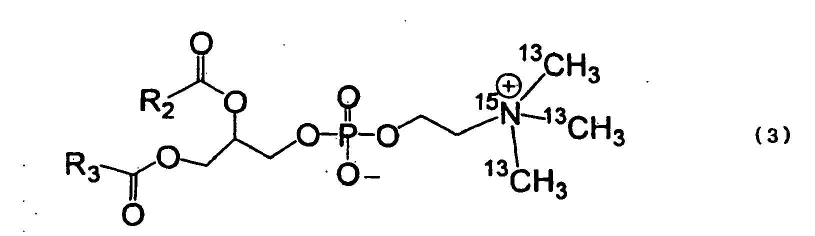 wherein R₂ and R₃ each independently represents a saturated or unsaturated alkyl group having 10 or more and 20 or less carbon atoms.

7. A diagnostic agent containing a compound according to any of claims 1 to 6 in an amount more than natural abundance ratio of its non-isotope labeled compound.

8. The diagnostic agent according to claim 7 which is an MR contrast agent.

9. A nuclear magnetic resonance analysis method wherein presence of the compound according to any of claims 1 so 6 it detected utilizing magnetic coherence transfer between nuclei in the ¹H-¹³C-¹⁵N bond of the compound.

10. A nuclear magnetic resonance imaging method wherein position of the compound according to any of claims 1 to 6 in a sample is detected by the nuclear magnetic resonance analysis method utilizing magnetic coherence transfer between nuclei in the ¹H-¹³C-¹⁵N bond of the compound.

11. A mass spectrometry method comprising a step of calculating the abundance ratio of the compound according to any of claims 1 to 6 to the other isotope compounds of the compound.

12. A mass spectrometry imaging method to obtain information on position where the compound according to any of claims 1 to 6 is present in a sample by calculating the abundance ratio of the compound according to any of claims 1 to 6 to the other isotope compounds of the compound.

## Patentansprüche

1. Verbindung, dargestellt durch die folgende allgemeine Formel (1): 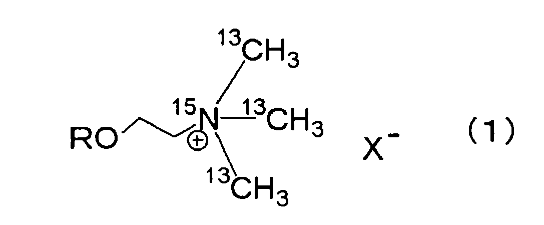 worin bedeuten
R: eine beliebige einwertige Gruppe und
X⁻: ein einwertiges Anion, vorausgesetzt, dass X⁻ fehlen kann, wenn R eine negative Ladung hat.

2. Verbindung nach Anspruch 1, wobei
die Verbindung vorliegt als ein markiertes Cholin mit einer Struktur, die dargestellt ist durch die folgende allgemeine Formel (2):

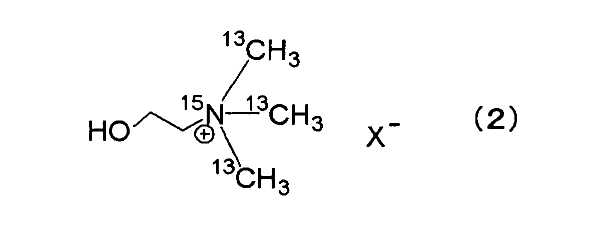

3. Verbindung nach Anspruch 1, wobei
R ein anderes als ein Wasserstoff-Atom ist.

4. Verbindung nach Anspruch 3, wobei
R vorliegt als Alkylcarbonylgruppe mit 2 oder mehr und 4 oder weniger Kohlenstoffatomen, oder das RO ein Phosphatrest ist.

5. Verbindung nach einem der Ansprüche 1 bis 4, wobei das X⁻ vorliegt als irgendein Ion, nämlich als ein Fluorid-Ion, ein Chlorid-Ion, ein Bromid-Ion, ein Jodid-Ion, ein Hydroxid-Ion und ein Tartrat-Ion.

6. Verbindung nach Anspruch 4, wobei
die Verbindung eine Struktur hat, welche dargestellt ist durch die folgende allgemeine Formel (3): 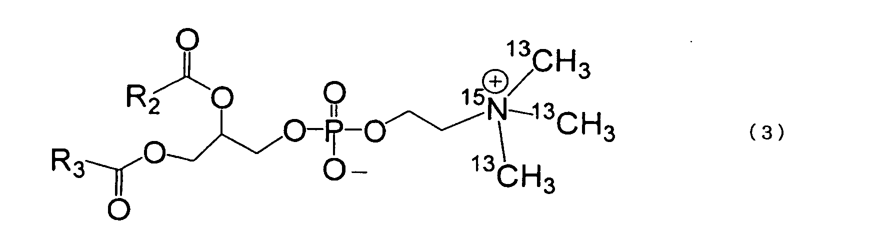 worin bedeuten
R₂ und R₃: je unabhängig eine gesättigte oder ungesättigte Alkylgruppe mit 10 oder mehr und 20 oder weniger Kohlenstoffatomen.

7. Diagnosemittel, enthaltend:
eine Verbindung nach einem der Ansprüche 1 bis 6 in einem Anteil größer als das natürliche Häufigkeitsverhältnis ihrer unmarkierten Isotopverbindung.

8. Diagnosemittel nach Anspruch 7, welches vorliegt als ein Kontrastmittel für NMR oder MRI.

9. Magnetisches Nuklearresonanzanalyseverfahren, wobei die Gegenwart der Verbindung nach einem der Ansprüche 1 bis 6 nachgewiesen wird unter Verwendung eines magnetischen Kohärenztransfers zwischen Nuklei in der ¹H-¹³C-¹⁵N-Bindung der Verbindung.

10. Magnetisches Nuklearresonanzabbildungsverfahren, wobei die Position der Verbindung nach einem der Ansprüche 1 bis 6 in einer Probe nachgewiesen wird durch das magnetische Nuklearresonanzanalyseverfahren unter Verwendung eines magnetischen Kohärenztransfers zwischen Nuklei in der ¹H-¹³C-¹⁵N-Bindung der Verbindung.

11. Massenspektrometrieverfahren, umfassend:
einen Schritt zum Berechnen des Häufigkeitsverhältnisses der Verbindung von einem der Ansprüche 1 bis 6 zu den anderen Isotopverbindungen der Verbindung.

12. Massenspektrometrieabbildungsverfahren zum Erhalt einer Information über eine Position, wo die Verbindung nach einem der Ansprüche 1 bis 6 in einer Probe vorhanden ist, durch Berechnen des Häufigkeitsverhältnisses von der Verbindung von einem der Ansprüche 1 bis 6 zu den anderen Isotopverbindungen der Verbindung.

## Revendications

1. Composé représenté par la formule générale (1) suivante,
dans laquelle R représente un groupe monovalent arbitraire et X⁻ représente un anion monovalent, sous réserve que X⁻ puisse être absent lorsque R a une charge négative.

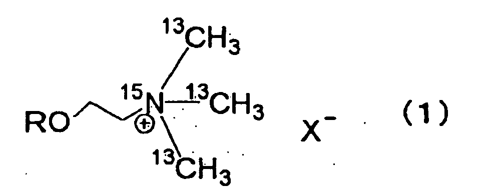

2. Composé suivant la revendication 1, ledit composé étant une choline marquée ayant une structure représentée par la formule générale (2) suivante.

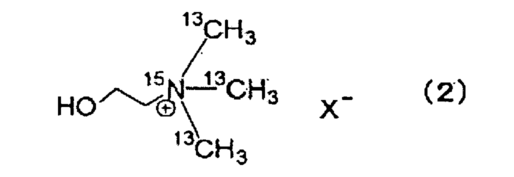

3. Composé suivant la revendication 1, dans lequel le groupe R est autre qu'un atome d'hydrogène.

4. Composé suivant la revendication 3, dans lequel le groupe R est un groupe alkylcarbonyle ayant 2 ou plus de 2 et 4 ou moins de 4 atomes de carbone ou bien le groupe RO est un résidu phosphate.

5. Composé suivant l'une quelconque des revendications 1 à 4, dans lequel X⁻ est n'importe lequel d'un ion fluorure, d'un ion chlorure, d'un ion bromure, d'un ion iodure, d'un ion hydroxyde et d'un ion tartrate.

6. Composé suivant la revendication 4, ledit composé ayant une structure représentée par la formule générale (3) suivante, 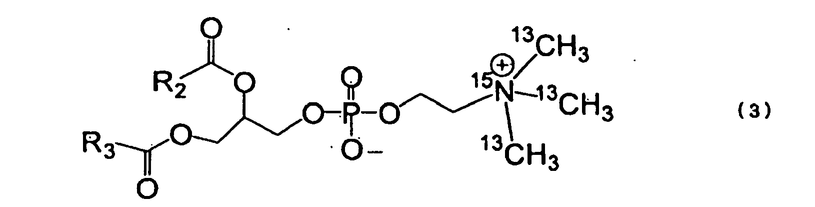 dans laquelle R₂ et R₃ représentent chacun indépendamment un groupe alkyle saturé ou insaturé ayant 10 ou plus de 10 et 20 ou moins de 20 atomes de carbone.

7. Agent de diagnostic contenant un composé suivant l'une quelconque des revendications 1 à 6 en une quantité supérieure à la proportion d'abondance naturelle du composé correspondant non marqué avec un isotope.

8. Agent de diagnostic suivant la revendication 7, qui est un agent de contraste pour résonance magnétique nucléaire (RMN) ou imagerie à résonance magnétique (IRM).

9. Méthode d'analyse par résonance magnétique nucléaire, dans laquelle la présence du composé suivant l'une quelconque des revendications 1 à 6 est découverte en utilisant le transfert de cohérence magnétique entre des noyaux dans la liaison ¹H-¹³C-¹⁵N du composé.

10. Méthode d'imagerie par résonance magnétique nucléaire, dans laquelle la position du composé suivant l'une quelconque des revendications 1 à 6 dans un échantillon est découverte par une méthode d'analyse par résonance magnétique nucléaire utilisant le transfert de cohérence magnétique entre des noyaux dans la liaison ¹H-¹³C-¹⁵N du composé.

11. Méthode de spectrométrie de masse, comprenant une étape de calcul du rapport d'abondance du composé suivant l'une quelconque des revendications 1 à 6 aux autres composés isotopiques correspondants.

12. Méthode d'imagerie par spectrométrie de masse pour obtenir une information sur la position où le composé suivant l'une quelconque des revendications 1 à 6 est présent dans un échantillon en calculant le rapport d'abondance du composé suivant l'une quelconque des revendications 1 à 6 aux autres composés isotopiques correspondants.
